## Europäisches Patentamt

(19) **European Patent Office**

**Office européen des brevets**

(11) Publication number: **0 284 224 B1**

(12) # EUROPEAN PATENT SPECIFICATION

(45) Date of publication of patent specification :
21.08.91 Bulletin 91/34

(51) Int. Cl.⁵ : **A61F 5/453, A61F 5/451**

(21) Application number : **88301852.5**

(22) Date of filing : **03.03.88**

(54) **External male incontinence device.**

(30) Priority : **04.03.87 US 21464**

(43) Date of publication of application :
**28.09.88 Bulletin 88/39**

(45) Publication of the grant of the patent :
**21.08.91 Bulletin 91/34**

(84) Designated Contracting States :
**AT BE CH DE ES FR GB GR IT LI LU NL SE**

(56) References cited :
**US-A- 4 540 409**
**US-A- 4 589 874**

(73) Proprietor : **SHERWOOD MEDICAL COMPANY**
**1831 Olive Street**
**St. Louis, MO 63103 (US)**

(72) Inventor : **Campion, Terese Ann**
**122 Chestnut Street-Apt. 306**
**Springfield Massachusetts 01103 (US)**
Inventor : **Sawatani, Hitoshi**
**No. 18-17, 2-chome Takadono**
**Asahi-ku Osaka (JP)**

(74) Representative : **Porter, Graham Ronald et al**
**c/o Wyeth Laboratories Huntercombe Lane**
**South**
**Taplow Maidenhead Berkshire SL6 0PH (GB)**

## Description

The present invention relates to an external male incontinence catheter and an applicator therefor.

Male incontinence catheters are well known and generally comprise an elastic sheath for surrounding the glans and shaft of the penis, the distal end of the sheath having an outlet which is connected via a drain tube to a urine collection container.

Problems with these catheters can arise during application to the penis. For example, when applying a sheath-type catheter having an adhesive-coated inner surface, care must be taken to ensure that the adhesive surface does not contact itself and that the sheath is evenly applied, without creating pockets-,voids or wrinkles. U.S. Patent 4,540,409 discloses a sheath-type external male catheter having a portion of its internal surface coated with an adhesive material and for use with a tubular applicator. The applicator is in the form of a relatively rigid open-ended, tapered tube, the length of which is less than the length of the fully extended sheath, and the diameter of which is slightly greater than the maximum inside diameter of the unstretched sheath. Before use, the distal end of the sheath and its outlet and drainage tube are disposed within the tube.

In use the cylindrical body portion of the sheath is everted over a portion of the outside surface of the applicator tube. So arranged, the outside surface of the sheath contacts the outside surface of the applicator tube, with the inner-side of the sheath, i.e., the side having the adhesive coating facing outwards and hence not capable of touching itself. In applying the catheter, the portion of the sheath which is not everted, i.e. the distal end, is placed over the glans and the applicator tube is moved along the penile shaft, thereby causing the everted portion of the sheath to revert so that the inner surface of the sheath contacts the penile shaft and the adhesive coating thereon secures the sheath in place.

While the aforementioned applicator tube tends to avoid the drawback associated with the adhesive-coated surfaces coming together, it has been found that the force required to slide the applicator in the reverting step is quite large and likely to tear the thin elastic sheath. Moreover, as a result of the force required to apply the catheter and the friction between the applicator and the penile shaft, there is an increased potential for the patient to experience an erection, a condition which is exacerbated by the presence of the tube applicator, which must be slid down the swollen shaft and glans of the penis for removal. Finally, this prior art device generally requires the additional processing step of providing a lubricant on the interior surface of the catheter to facilitate the application of the catheter sheath to the penis. The presence of a lubricant adds to the problems associated with the application of the external catheter.

According to the invention there is provided an external male incontinence device comprising an elastic sheath for application to the penis and having a drain outlet, and a relatively rigid applicator ring, the proximal end of the sheath being everted over the distal end and said ring being a snug fit around the open end of the everted sheath.

The applicator ring fits closely around the adjacent portion of the everted sheath but does not stretch or otherwise distort the sheath. The open end of the device is applied to the tip of the penis and the sheath unrolled as the ring is moved down the penile shaft. The ring is much less likely to tear the sheath because of the rolling action and a lubricant is not needed. The likelihood of erection is also substantially reduced.

A skin compatible adhesive may be applied to the outer surface of the everted sheath and is preferably covered by the ring. In a specific embodiment the adhesive is a relatively narrow circumferential band which peels from the ring onto the penis as the sheath unrolls. The invention gives precise application of the adhesive band which this reduces both the amount of adhesive necessary and the likelihood of wrinkling & pocketing.

The ring may be axially split to allow easy removal after use thus further reducing the chance of causing discomfort to the patient. An external flange maybe provided on the ring to give the user a convenient hand hold.

In the preferred embodiment the proximal end of the sheath is longer than the axial width of the applicator ring, the excess length being reverted over a distal portion of the ring. An external circumferential groove in the ring provides a seat for the reverted portion of the sheath.

The distal end of the sheath is preferably of thicker material than the proximal end so as to be relatively shape retaining.

Other features of the invention will be apparent from the following description of a preferred embodiment illustrated shown by way of example only in the accompany drawings in which :

Figure 1 is a perspective view of a catheter according to the present invention ;

Figure 2 is a perspective view of an applicator ring for use with the catheter of Figure 1.

Figure 3 is a perspective view illustrating the catheter and applicator ring in combination, with an everted sheath portion extending below the ring ;

Figure 4 is an enlarged perspective view, partially broken away,of the catheter and applicator ring in combination and as they would appear prior to use.

Figure 5 is an axial section through the catheter and applicator ring combination illustrated in Figure 4.

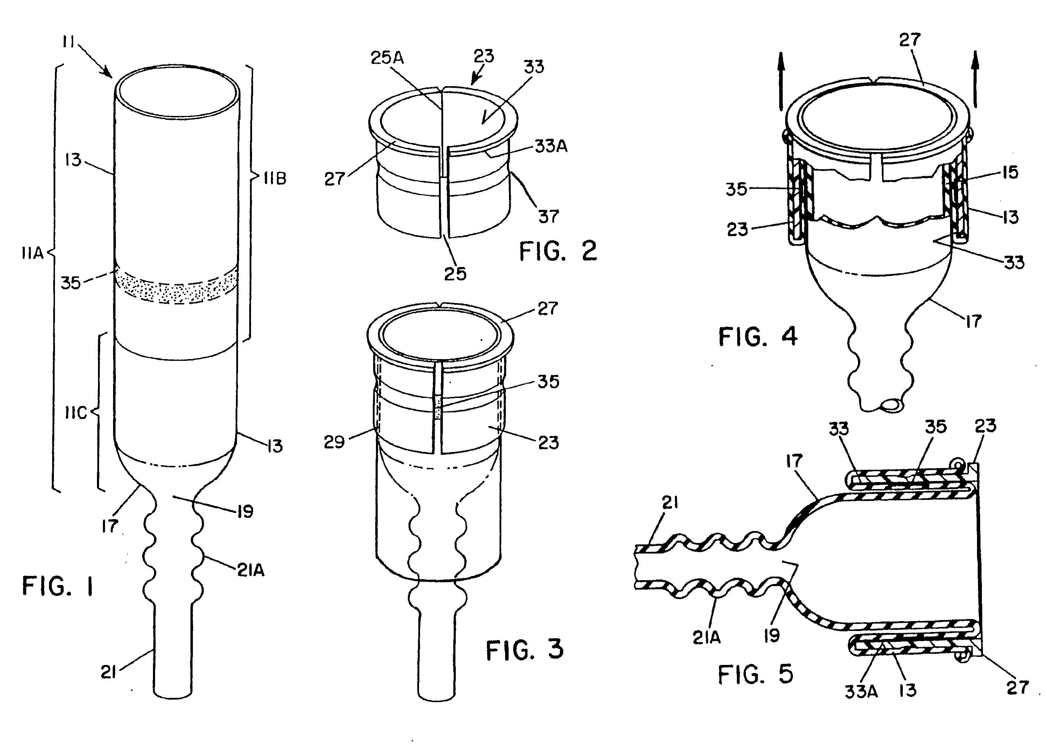

The external male incontinence catheter shown in Figure 1 comprises one of the two principal elements of the present invention. The other element is the applicator ring, illustrated in Figure 2.

With reference to Figure 1 a catheter 11 is made of a suitable soft, highly elastic, natural or synthetic rubber. Natural latex is preferred but alternative elastomers having similar properties may be used. The catheter includes an elongate, generally cylindrical sheath 11A having a proximal portion 11B and an integral distal portion 11C.

The distal portion 11C is constructed so as to be relatively shape retaining and in the preferred embodiment the wall thickness of the distal portion is substantially greater than the wall thickness of the proximal portion 11B.

The end 17 of the distal sheath portion 11C tapers gradually inward to an outlet 19 and drain tube 21 as shown. The proximal end of drain tube 21 has convolutions 21A to give additional flexibility. The distal sheath portion 11C, outlet 19 and convolutions 21A are integral and made from the same thickness of material.

The thickness of the upper portion 11B may range from 0.075-0.25 mm (0.003 to 0.010 inches) while the thickness of the lower portion 11C may be approximately 0.75 mm (0.030 inches). The thickness of tube 21 is approximately 1.0-1.5 mm (0.040 to 0.060 inches).

The catheter 11 is provided with an internal adhesive band 35 to secure the sheath to the penis. The band can be any suitable grade of skin compatible, pressure sensitive adhesive and in the preferred embodiment is a hypo-allergic acrylic adhesive. The position of band 35 is such that, after application, the adhesive contacts the penis just below the corona of the glans.

The applicator ring 23 is generally cylindrical and open at both ends. An axially extending slot 25 permits the ring to be opened about a hinge line 25A. At the proximal end the ring has an outwardly extending flange 27 which is cut away adjacent the hinge line. A circumferential groove 37 extends around the ring midway between the ends as shown.

Slot 25 facilitates manufacture of the ring, and assembly and removal of the ring from the catheter as will be further described below. Groove 37 provides a seat for the catheter in the assembled condition.

Ring 23 may be made from any suitable material, for example polypropylene, which gives a smooth relatively rigid ring structure.

In the assembled condition the internal diameter of ring 23 is substantially equal to the adjacent portion of the sheath so that the ring is a snug fit and surrounds the sheath without distorting the shape thereof.

The sheath and applicator ring are assembled as follows :

Proximal portion 11B is first everted. In this position, the outer wall 13 of the proximal portion 11B contacts the outer wall of the distal portion 11C, with the inner wall 15 of the proximal portion 11B encircling the convolutions 21A. Adhesive band 35 is applied to the surface of the everted proximal portion 11B, which is facing outward. Ring 23 is opened and placed about the everted sheath portion, so that the inner surface of ring 23 contacts the adhesive band 35. The device in this condition is shown in Figure 3. Thereafter, the everted portion 11B which encircles the convolutions 21A is reverted over the outer surface of ring 23 as shown in Figs. 4 and 5. Groove 37 provides a seat for the outermost portion of the sheath as shown in Fig. 5.

Application of the device is effected smoothly and simply by placing the opening of catheter portion 11C over the glans of the penis ; lightly gripping flange 27 and moving the applicator ring 23 along the penile shaft so as to cause sheath 11 to unroll, whereby adhesive band 35 peels from ring 23 onto the penis.

Ring 23 may then be opened for easy removal from around the shaft of the penis.

While the foregoing discloses an embodiment of the invention in detail, it will be understood by those skilled in the art that various aspects of the present invention may be modified without departing from the scope of the invention.

## Claims

1. An external male incontinence device comprising an elastic sheath (11) for application to the penis and having a drain outlet, (19) and a relatively rigid applicator ring (23), the proximal end (11B) of the sheath being everted over the distal end (11C) and said ring (23) being a snug fit around the open end of the everted sheath.

2. A device according to Claim 1 wherein a skin compatible adhesive is applied to the outer surface of said everted sheath.

3. A device according to any Claim 1 or Claim 2 wherein said ring (23) has an outwardly extending flange (27) at the proximal end thereof.

4. A device according to any preceding Claim wherein said ring (23) is axially split.

5. A device according to Claim 4 wherein said adhesive is applied as a circumferential band (35) and covered by said ring.

6. A device according to any preceding Claim wherein the proximal end of said sheath is longer than the axial width of said ring, the excess length being reverted over a distal portion of said ring.

7. A device according to Claim 6 wherein said ring (23) has an external circumferential groove (37) to provide a seat for said excess length.

8. A device according to any preceding Claim

EP 0 284 224 B1

wherein the distal end (11C) of said sheath is of thicker material than said proximal end (11B) so as to be relatively shape retaining.

9. A device according to any preceding Claim wherein the distal end (11C) of said sheath tapers inwardly to form an integral drain tube (21).

10. A device according to Claim 9 wherein the proximal end of said tube (21) is convoluted.

**Patentansprüche**

1. Vorrichtung zur äußeren Anwendung bei männlicher Inkontinenz, die eine elastische Hülse (11) zum Anlegen an den Penis mit einer Ablauföffnung, (19) und einen relativ steifen Anlege-Ring (23) umfaßt, wobei das proximale Ende (11B) der Hülse über das distale Ende (11C) gestülpt ist, und der Ring (23) einen Pass-Sitz um das offene Ende der umgestülpten Hülse darstellt.

2. Vorrichtung nach Anspruch 1, bei der ein hautverträgliches Klebemittel auf die Außenfläche der umgestülpten Hülse aufgebracht ist.

3. Vorrichtung nach einem der Ansprüche 1 oder 2, bei der der Ring (23) an seinem proximalen Ende einen nach außen gerichteten Flansch (27) aufweist.

4. Vorrichtung nach einem der vorhergehenden Ansprüche, bei der der Ring (23) axial gespalten ist.

5. Vorrichtung nach Anspruch 4, bei der das Klebemittel als umlaufendes Band (35) aufgebracht und von dem Ring überdeckt ist.

6. Vorrichtung nach einem der vorhergehenden Ansprüche, bei der das proximale Ende der Hülse länger als die Breite des Rings in Axialrichtung ist, und die Überlänge über einen distalen Teil des Rings umgeschlagen ist.

7. Vorrichtung nach Anspruch 6, bei der der Ring (23) eine äußere umlaufende Einkerbung (37) aufweist, die der Überlänge einen Sitz verleiht.

8. Vorrichtung nach einem der vorhergehenden Ansprüche, bei der das distale Ende (11C) der Hülse aus dickerem Material als das proximale Ende (11B) ist, um so relativ formstabil zu sein.

9. Vorrichtung nach einem der vorhergehenden Ansprüche, bei der sich das distale Ende (11C) der Hülse nach innen verjüngt, um eine integrierte Ablaufröhre (21) zu bilden.

10. Vorrichtung nach Anspruch 9, bei der das proximale Ende der Röhre (21) gewellt ist.

**Revendications**

1. Dispositif externe pour l'incontinence masculine, comprenant une gaine élastique (11) à appliquer sur le pénis et comportant un orifice de sortie de drainage (19) et un anneau applicateur relativement rigide (23), l'extrémité proximale (11B) de la gaine étant retournée à l'envers par-dessus l'extrémité distale (11C) et l'anneau (23) étant étroitement ajusté autour de l'extrémité ouverte de la gaine retournée à l'envers.

2. Dispositif suivant la revendication 1, dans lequel un adhésif compatible avec la peau est appliqué sur la surface extérieure de la gaine retournée à l'envers.

3. Dispositif suivant la revendication 1 ou 2, dans lequel l'anneau (23) comporte une collerette extérieure (27), à son extrémité proximale.

4. Dispositif suivant l'une quelconque des revendications précédentes, dans lequel l'anneau (23) est divisé axialement.

5. Dispositif suivant la revendication 4, dans lequel l'adhésif est appliqué sous la forme d'une bande circonférentielle (35) et est recouvert par l'anneau.

6. Dispositif suivant l'une quelconque des revendications précédentes, dans lequel l'extrémité proximale de la gaine est plus longue que la largeur axiale de l'anneau, la longueur en excès étant retournée à l'endroit sur une partie distale de l'anneau.

7. Dispositif suivant la revendication 6, dans lequel l'anneau (23) présente une gorge circonférentielle externe (37) formant un siège pour la longueur en excès.

8. Dispositif suivant l'une quelconque des revendications précédentes, dans lequel l'extrémité distale (11C) de la gaine est faite d'une matière plus épaisse que l'extrémité proximale (11B) de manière à conserver relativement sa forme.

9. Dispositif suivant l'une quelconque des revendications précédentes, dans lequel l'extrémité distale (11C) de la gaine va en diminuant vers l'intérieur pour former un tube de drainage (21) qui en fait partie intégrante.

10. Dispositif suivant la revendication 9, dans lequel l'extrémité proximale du tube (21) est annelée.

FIG. 1

FIG. 2

FIG. 3

FIG. 4

FIG. 5